# EUROPEAN PATENT APPLICATION

(11) **EP 2 233 131 A1**
(43) Date of publication of application: **29.09.2010**
(21) Application number: 09155908.8
(22) Date of filing: 23.03.2009
(51) Int. Cl.: A61K 9/20, A61K 31/00

(54) **Pharmaceutical composition containing levodopa, entacapone and carbidopa**

(71) Applicant: Laboratorios Lesvi, S.L., 08970 Sant Joan Despi (ES)
(72) Inventor: Díez Martín, Ignacio, 08970 Sant Joan Despí (ES); Pablo Alba, Pablo, 08970 Sant Joan Despí (ES); Úbeda Pérez, Carmen, 08970 Sant Joan Despí (ES)

(57) **Abstract**

The present invention relates to a pharmaceutical composition of entacapone, levodopa and carbidopa or phamiaceutically aceptable salts or hydrates thereof characterized in that it comprises a first mixture of entacapone and levodopa and a second mixture of levodopa and carbidopa. The invention also relates to a preparation method of said composition.

## Description

### FIELD OF THE INVENTION

The present invention relates to a new pharmaceutical composition of entacapone, levodopa and carbidopa or pharmaceutically acceptable salts or hydrates thereof **characterized in that** it comprises a first mixture of entacapone and levodopa and a second mixture of levodopa and carbidopa. The invention also relates to a preparation method of said composition.

### BACKGROUND OF THE INVENTION

Entacapone or (E)-2-cyano-3-(3,4-dihydroxy-5-nitrophenyl)-N,N-diethyl-2-propenamide is described in U.S. Patent No. 5,446,194 as a cathecol-O-methyltransferase (COMT) inhibitor. An oral pharmaceutical composition containing entacapone and crosscarmelose sodium is marketed in Europe as COMTESS® and COMTAN®. These products are indicated as an adjunct to standard preparations of levodopa/benserazide or levodopa/carbidopa for use in patients with Parkinson's disease and end-of-dose motor fluctuations who cannot be stabilized on those combinations.

Levodopa and carbidopa are commonly used drugs in the treatment of Parkinson's disease. Combination of levodopa and carbidopa in form of tablets is marketed under the trademark SINEMET®.

Combination of levodopa, carbidopa and entacapone is marketed under the trademark STALEVO® to Orion and it is indicated for the treatment of patients with Parkinson's disease and end-of-dose motor fluctuations not stabilized on levodopa/dopa decarboxylase (DDC) inhibitor treatment.

European patent EP 1 189 608 B1 describes a pharmaceutical composition comprising entacapone, levodopa and carbidopa or pharmaceutically acceptable salts or hydrates thereof wherein carbidopa is added separately to the composition, e.g. entacapone and levodopa, together with (an) excipient(s). The patent indicates that, commonly used excipients (microcrystalline cellulose, and/or surface active agents such as polyethylene glycol, polysorbate and sodium lauryl sulphate and/or silica) are not suitable to be used in the formulation because they are incompatible with the drug combination. Moreover, formulations where all three ingredients are wet granulated together provided insufficient absorption of carbidopa. It has been observed that carbidopa may undergo Micheal addition to entacapone to form additional degradation products. Degradation by this pathway is minimized by tablet manufacturing process, in which carbidopa and entacapone are separated. WO2008/053297 discloses formulations where entacapone is separated from levodopa and carbidopa. This application discloses tablet in a tablet, bilayered tablets, and other formulations.

Therefore, there is still a need for developing pharmaceutical compositions which incorporate levodopa, carbidopa and entacapone or their pharmaceutical acceptable salts and methods for preparing such compositions with an improved physical stability and without the active ingredient release properties being affected and efficient and industrial feasible methods for their preparation.

### BRIEF DESCRIPTION OF THE INVENTION

The aim of the present invention is to provide pharmaceutical compositions containing levodopa, carbidopa and entacapone or a pharmaceutically acceptable salts or hydrates thereof for oral administration having agood dissolution profile and an improved physical stability without affecting the release profile of said active ingredients. In addition, it is an aim of the present invention to provide a process for preparing said pharmaceutical compositions, particularly tablets, by means of a process that can be applied at an industrial level with low energy costs and which does not subject the active ingredient to aggressive formulation conditions which can entail the loss of stability of the product.

The combination Entacapone-Levodopa-Carbidopa (Stalevo ®) is commercialized in four fixed dosages:

| | | | | |
|---|---|---|---|---|
| **Entacapone** | 200 mg | 200 mg | 200 mg | 200 mg |
| **Levodopa** | 50 mg | 100 mg | 150 mg | 200 mg |
| **Carbidopa** | 12.5 mg | 25 mg | 37.5 mg | 50 mg |

The authors of the present invention have found that the use of a first mixture of entacapone and a substantial amount of levodopa, in fixed amounts and their separate addition to a second mixture of levodopa and carbidopa, in a variable proportion depending on the dosage form to be prepared, provides a simple method for the preparation of oral pharmaceutical compositions. By this means, the physical stability is improved without affecting the dissolution properties of the oral compositions, making them suitable for therapeutic use. Alternatively, the second mixture of levodopa and carbidopa is prepared in fixed amounts and the remaining amount of levodopa is added separately. The pharmaceutical composition can be in form of tablets or capsules.

Accordingly, a first aspect of the present invention is a solid pharmaceutical composition for oral intake comprising entacapone, levodopa and carbidopa or pharmaceutically acceptable salts or hydrates thereof **characterized in that** it comprises:
a) A first mixture wherein 10 to 75% of the total amount of levodopa is mixed with entacapone, optionally in the presence of pharmaceutical acceptable excipients ;
b) A second mixture wherein from 25 to 90% of the total amount of levodopa is mixed with carbidopa, optionally in the presence of pharmaceutical acceptable excipients;
c) Optionally, a third mixture of remaining levodopa and pharmaceutical acceptable excipients.

The first, second and third mixtures can be in the form of powder, granules, coated granules, pellets, etc, independently of the final form of the pharmaceutical composition, which can be tablets, optionally coated or capsules; with the proviso that the first and second mixture can not be both in the form of powder. Optionally, the remaining amount of levodopa is mixed with pharmaceutical excipients in the form of powder, granules or coated granules that are mixed with the first and second mixture.

Preferably, the ratio entacapone-levodopa in the first mixture is from 1:0.05 to 1:0.75 by weight. More preferably, the ratio is 1:0.1 to 1:0.2.

In a first embodiment, the pharmaceutical composition is in the form of a tablet.

In another embodiment, the pharmaceutical composition is in the form of a capsule.

According to a particular embodiment, levodopa and carbidopa are together in the form of granules. Optionally, these granules can be coated. In another embodiment, levodopa and carbidopa are in the form of powders.

In a particular embodiment, entacapone and levodopa mixture are together in form of powders. In a particular embodiment, entacapone and levodopa are together in form of granules, optionally coated.

A second aspect of the present invention is a method for preparing a solid oral pharmaceutical composition according as defined above, comprising the stages of:
a) providing a first mixture of entacapone or a pharmaceutically acceptable salt thereof and a substantial amount of levodopa and, optionally together with excipients;
b) providing a second mixture of levodopa and carbidopa or pharmaceutically acceptable salts thereof optionally together with excipients;
c) Optionally, mixing levodopa with excipients.
d) Mixing the mixtures obtained in steps a),b) and optionally c)
e) Optionally, adding extragranular excipients
f) Filling the mixture into a capsule or compressing into a tablet
g) Optionally, coating the tablet.

### DETAILED DESCRIPTION OF THE INVENTION

In the present invention, "entacapone" is understood to be the compound entacapone or a pharmaceutically acceptable salt, hydrate o solvate thereof. Entacapone may be incorporated in the composition in any solid form either as a free compound or as a hydrate or solvate.

Unless otherwise indicated, as "levodopa" and "carbidopa" it is understood the compounds levodopa and carbidopa or their pharmaceutically acceptable salts, hydrates or solvates. Carbidopa is preferably used in form of monohydrate.

Entacapone, levodopa and carbidopa are preferably in pharmaceutically acceptable or substantially pure form. By pharmaceutically acceptable form is meant, *inter alia,* having a pharmaceutically acceptable level of purity excluding normal pharmaceutical additives such as diluents and carriers, and including no material considered toxic at normal dosage levels. Purity levels for the drugs substances are preferably above 50%, more preferably above 70%, most preferably above 90%. In a preferred embodiment it is above 95% of the drugs or of their salts, solvates, hydrates or prodrugs.

The invention provides a solid oral pharmaceutical composition of entacapone, levodopa and carbidopa **characterized in that** it comprises:
a) A first mixture wherein 10 to 75% of the total amount of levodopa is mixed with entacapone, optionally in the presence of pharmaceutical acceptable excipients;
b) A second mixture wherein from 25 to 90% of the total amount of levodopa is mixed with carbidopa, optionally in the presence of pharmaceutical acceptable excipients.
c) Optionally, a third mixture of containing from 0 to 60% of the total amount of levodopa and pharmaceutical acceptable excipients;
with the proviso that the first and second mixtures can not be at the same time in form of powder.

The first mixture comprises from 10 to 75% of the total amount of levodopa that is mixed with all the entacapone present in the formulation. Preferably, it contains from 15 to 60% of the total amount of levodopa. This mixture can be in the form of powder, granules, coated granules, pellets, etc, independently of the final form of the pharmaceutical composition, optionally including at least one binder agent, and/or one diluent agent and/or a disintegrant agent.

Preferably, the ratio entacapone-levodopa in the first mixture is from 1:0.05 to 1:0.75 by weight. More preferably, the ratio is 1:0.1 to 1:0.2. In a preferred embodiment, 30 mg of levodopa are mixed with 200 mg of entacapone, optionally in the presence of pharmaceutical acceptable ingredients to form the first mixture. This first mixture can be used to prepare all the strengths of the composition that are available in the market.

Entacapone is a very poor soluble compound (80 µg/ml pH≤5; Drug Development Research, 2000, 49(4) 238-244*)* and its bioavailability after oral administration is low and subject to a large individual variation. The solubility of entacapone can be increased with increased pH. However, it has been observed a substantial increase of the degradation products of the three active ingredients when entacapone, levodopa and carbidopa are dissolved in alkaline solutions.. It has surprisingly been observed that the incorporation of a substantial amount of levodopa in the mixture with entacapone enhances the solubility of this compound.

The second mixture comprises from 25 to 90% of the total amount of levodopa that is mixed with all the carbidopa present in the formulation. Preferably, it contains from 30 to 85% of the total amount of levodopa. This mixture can be in the form of powder, granules, coated granules, pellets, etc, independently of the final form of the pharmaceutical composition.

Optionally, a remaining amount of levodopa (from 0 to 60%) is mixed with pharmaceutical excipients in the form of powder or granules , which are mixed with the first and second mixture.

In a particular embodiment, a second fixed mixture of levodopa and carbidopa is prepared once and used in the preparation of all strengths. Thus, for instance, levodopa and carbidopa monohydrate are mixed in a ratio from 0.3:1 to 1.5:1, more preferably 1:1 to 1.3:1, along with a sufficient amount of excipients. This second fixed mixture comprises from 30% to 70% of levodopa, more preferably, from 35 to 60% of levodopa, from 10 to 40% of carbidopa monohydrate, and 0-15% of binder, 0-20% diluent and 0-10% disintegrant. All these components are mixed together to obtain a second fixed mixture. Preferably, the components are mixed by a granulation process. This second fixed mixture is prepared in enough quantity to be used in the preparation of all strengths. Then, the required amount is used depending on the strengths, i.e. 10% of the total quantity prepared is used in the lowest strength, 20% in the second, 30% in the 150/25mg and 40% in the highest strength. If required, the remaining amount of levodopa is adjusted by means of a third mixture comprising levodopa and excipients.

This embodiment, simplifies the industrial manufacturing of the composition containing three active ingredients, since the mixtures in fixed proportions of the active ingredients are prepared once and used in all the strengths, reducing in this way the number of process steps. In a first embodiment, the final pharmaceutical composition is in the form of a tablet. Preferably, film coated tablets.

In another embodiment, the final pharmaceutical composition is in the form of a capsule.

By the term "granules" it is understood a core which comprises the active ingredients and, which may optionally comprise at least one binder agent, one diluent agent and/or a disintegrant agent. In addition, the granules may optionally be coated with a film coating formulation or fatty excipients.

By the term "powder" it is understood a direct mixture of all active and inactive ingredients which comprises the active ingredients and, which may optionally comprise at least one binder agent, one diluent agent and/or a disintegrant agent.

The binder agent included in the core of the granules or in the powder is selected from povidone, cornstarch, hydroxypropylcellulose, hydroxypropylmethyl cellulose, methyl cellulose, pregelatinized starch, low-substituted hydroxypropyl cellulose, copovidone and mixtures thereof.

The diluent agent optionally included in the core of the granules or in the powder is preferably selected from microcrystalline cellulose, cellulose powdered, lactose monohydrate, anhydrous lactose, cornstarch and maltose. Examples of diluents which can be used in the invention include, without limitation, saccharides such as monosaccharides, oligosaccharides or polysaccharides, and/or their oxidised and/or reduced forms; ribose, lactose, maltose in its various forms, anhydrous, monohydrate, agglomerated forms or atomised forms; sugar alcohols such as mannitol, maltol, sorbitol, maltitol, xylitol, and erythritol, cellulose powder, microcrystalline cellulose, silified microcrystalline cellulose or derivatives of cellulose modified chemically, such as hydroxypropyl cellulose, hydroxypropylmethyl cellulose; isomalt, starch, sucrose, or mixtures thereof.

In a particular embodiment, it is possible to prepare granules of entacapone and levodopa, and granules of carbidopa and levodopa using only one intragranular excipient which could act as diluent, binder and disintegrant. Such excipient can be microcrystalline cellulose, cellulose powder or starch. In a preferred embodiment, maize starch is used.

The disintegrant agent optionally included in the core of the granule or in the powder is selected from natural starches, such as maize starch and potato starch; directly compressible starches such as starch 1500; modified or pregelatinized starches such as carboxymethylstarches and sodium starch glycolate; natural or chemically-modified cellulose, especially crosslinked sodium carboxymethyl cellulose (crosscarmelose sodium) or low substituted hydroxypropyl cellulose; microcrystalline cellulose; gum, especially agar gum, and guar gum; alginic acid or salts thereof; acetates and citrates; sugars (especially lactose, mannitol and sorbitol); synthetic polymers such as cross-linked polyvinylpyrrolidones, specially crospovidone.

In a particular embodiment, the granules prepared can be coated using fatty excipients such as gliceryl behenate, gliceryl palmitostearate, or cellulose derivatives such as hydroxypropylmethyl cellulose.

The film coating formulation used to coat the granule may comprise at least one polymer and/or one plasticizer or/and a colorant. Accordingly, polymers usable in the film coating formulation are cellulose ethers, particularly, hydroxypropyl cellulose, hydroxypropyl methylcellulose, methylcellulose and mixtures thereof or, alternatively, acrylics, such as methacrylate and methyl methacrylate copolymers or vinyls such as polyvinyl alcohol. Examples of plasticizers usable in the film coating formulation are triethyl citrate, triacetin, propylene glycol, glycerol, macrogol, and mixtures thereof. Examples of colorants usable in the film coating formulation are titanium dioxide, yellow and red iron oxides and the like.

When the first, the second or the third mixtures are in form of granules, they can be prepared by any method known in the state of the art.

In a particular embodiment, when the first mixture is in form of granules, these are prepared by granulation of entacapone and a 10 to 75% of the total amount of levodopa and optionally with a binder, a diluent agent and/or a disintegrant agent, optionally followed by a coating process. The process may comprise wet, dry and hot melt granulation. The wet granulation process is preferred.

One of the major problems when handling entacapone in the manufacture of pharmaceutical compositions is the difficulty in removing coloured residues from the machinery because of its low solubility in usual solvents, giving problems of cross-contamination in multipurpose equipment which is unacceptable for pharmaceutical purposes. Furthermore, entacapone has an intense yellow color which may stain porous materials such as textiles, fabrics or plastics.

Advantageously, this fixed mixture of levodopa and entacapone can be used in the preparation of the four dosage forms commercially available.

Thus, the present invention provides a mixture of entacapone and a substantial amount of levodopa, which can be prepared once and used in the preparation of the four dosages, reducing the number of the process steps. It is especially important to note that the number of process steps in which entacapone is manipulated is significantly reduced and therefore, the cleaning operations are reduced and the risk of cross-contamination is also reduced.

In a particular embodiment, when the second mixture is in form of granules, these are prepared by granulation of carbidopa and a 25 to 90 % of the total amount of levodopa and optionally with a binder, a diluent agent and/or a disintegrant agent, optionally followed by a coating process. The process may comprise wet, dry and hot melt granulation. The wet granulation process is preferred. Optionally, a third mixture of levodopa (from 0 to 60% of the total amount) with pharmaceutical excipients is prepared in form of powder or granules.

Finally, the first and second mixture in form of granules, optionally coated, or in form of powder, with the proviso that the two mixtures can not be in form of powder at the same time, and optionally the third mixture, and optionally extragranular excipients, are compressed into a tablet. In a particular embodiment, the tablet is coated with a film forming agent. In an alternative embodiment, the first and second mixture in form of granules, optionally coated, or powder and optionally the third mixture are filled in a hard capsule.

Furthermore, it has been found that the present manufacturing process does not affect the dissolution profiles, which are comparable to commercial tablets of the combination. The present invention also provides smaller pharmaceutical compositions than the commercially available tablets. The weight of a tablet of Stalevo 200/150/37.5 is 720 mg whereas, the weight of tablets or capsules prepared according to the present invention is around 670 mg for the same dosage. Thus, the present invention provides smaller pharmaceutical compositions which are very convenient for patients suffering from Parkinson's disease that may have problems in swallowing.

The present invention provides a process for the preparation of a pharmaceutical composition comprising a mixture of levodopa, entacapone and carbidopa.

Accordingly, the process for preparing such a composition comprises the steps of:
a) providing a first mixture of entacapone or a pharmaceutically acceptable salt thereof and a substantial amount of levodopa and, optionally mixing it with at least one binder agent, and/or one diluent agent and/or a disintegrant agent;
b) Providing a second mixture of levodopa and carbidopa or pharmaceutically acceptable salts thereof optionally together with excipients.
c) Optionally, mixing levodopa with excipients.
d) Mixing the mixtures obtained in steps a),b) and c)
e) Optionally adding extragranular excipients
f) Filling the mixture into a capsule or compressing into a tablet
g) Optionally, coating the tablet.

The step a) consists of providing a first mixture containing all entacapone or a pharmaceutically acceptable salt thereof and a substantial amount of levodopa and optionally mixing it with a binder, a disintegrant and/or a diluent. The amount of levodopa in the mixture is comprised between 10 and 75% of the total amount of the levodopa in the formulation, preferably between 15 and 60%. Preferably, the ratio entacapone-levodopa in the first mixture is from 1:0.05 to 1:0.75 by weight. More preferably, the ratio is 1:0.1 to 1:0.2. Optionally mixing it with at least one binder agent, and/or one diluent agent and/or a disintegrant agent;

In a preferred embodiment, 30 mg of levodopa are mixed with 200 mg of entacapone, optionally in the presence of pharmaceutical acceptable ingredients to form the first mixture. This first mixture can be used to prepare all the strengths of the composition that are available in the market.

The amount of disintegrant to be optionally added is comprised between 1% and 20% by weight with respect to the total weight of the first mixture. Preferably, between 1% and 10% is added, more preferably between 2% and 10% of the total weight of the first mixture. The amount of diluent to be optionally added is comprised between 1% and 25% by weight with respect to the total weight of the first mixture, preferably between 2% and 20% of the total weight. The amount of binder to be optionally added to the mixture obtained in step a) is comprised between 1% and 15% by weight with respect to the total weight of the first mixture.

In a particular embodiment, the first mixture is further granulated. The granulation process may include the dry granulation, wet granulation or hot melt granulation. Wet granulation is preferred. In the wet granulation process, the solvent is added in an amount comprised between 2% and 50% by weight with respect to the weight of mixture to be granulated, which will serve to carry out the wet mixture.

Alternatively, the binder agent can be previously dissolved or suspended in said solvent and then added to entacapone and levodopa or to the mixture of entacapone, levodopa and excipients. As solvents for preparing the wet mixture, purified water, hydroalcoholic mixtures and alcohols can be used, being preferred the use of purified water as a solvent for the granulation of the mixture. This solvent is later eliminated from the composition by means of a drying step. Once the wet granules are obtained, they are subjected to a drying process to eliminate the solvent. Subsequently, the dried granules are calibrated by sieving or milling. Optionally, the sieved or milled granules are coated with film coating solution or with fatty excipients. The coating process can be carried out by any process known by a skilled person.

The step b) consists of providing a first mixture containing all carbidopa and a substantial amount of levodopa and optionally mixing it with a binder, a disintegrant and/or a diluent. The amount of levodopa in the mixture is comprised between 25 and 90% of the total amount of the levodopa in the formulation, preferably between 30 and 85%.

The amount of disintegrant to be optionally added is comprised between 1% and 20% by weight with respect to the total weight of the first mixture. Preferably, between 1% and 12% is added, more preferably between 2% and 10% of the total weight of the first mixture. The amount of diluent to be optionally added is comprised between 1% and 40% by weight with respect to the total weight of the second mixture, preferably between 5% and 20% of the total weight. The amount of binder to be optionally added to the mixture obtained in step b) is comprised between 1% and 15% by weight with respect to the total weight of the second mixture.

In a particular embodiment, the second mixture is further granulated. The granulation process may include the dry granulation, wet granulation or hot melt granulation. Wet granulation is preferred. In the wet granulation process, the solvent is added in an amount comprised between 2% and 50% by weight with respect to the weight of mixture to be granulated, which will serve to carry out the wet mixture. Alternatively, the binder agent can be previously dissolved or suspended in said solvent and then added tocarbidopa and levodopa or to the mixture of carbidopa and levodopa and excipients. As solvents for preparing the wet mixture, water, hydroalcoholic mixtures and alcohols can be used, being preferred the use of water as a solvent for the granulation of the mixture. This solvent is later eliminated from the composition by means of a drying step. Once the wet granules are obtained, they are subjected to a drying process to eliminate the solvent. Subsequently, the dried granules are calibrated by sieving or milling. Optionally, the sieved or milled granules are coated with film coating solution or fatty excipients. The coating process can be carried out by any process known by a skilled person.

Optionally, in the step c), levodopa in an amount from 0 to 60% of the total amount of levodopa is mixed with excipients in form of powder.

In a particular embodiment, the third mixture is further granulated. The granulation process may include the dry granulation, wet granulation or hot melt granulation. Wet granulation is preferred. In the wet granulation process, the solvent is added in an amount comprised between 2% and 50% by weight with respect to the weight of mixture to be granulated, which will serve to carry out the wet mixture. Alternatively, the binder agent can be previously dissolved or suspended in said solvent and then added to levodopa or to the mixture of levodopa and excipients. As solvents for preparing the wet mixture, water, hydroalcoholic mixtures and alcohols can be used, being preferred the use of water as a solvent for the granulation of the mixture. This solvent is later eliminated from the composition by means of a drying step. Once the wet granules are obtained, they are subjected to a drying process to eliminate the solvent. Subsequently, the dried granules are calibrated by sieving or milling. Optionally, the sieved or milled granules are coated with film coating solution or fatty excipients. The coating process can be carried out by any process known by a skilled person.

The amount of disintegrant to be optionally added is comprised between 1% and 20% by weight with respect to the total weight of the third mixture. Preferably, between 1% and 12% is added, more preferably between 2% and 10% of the total weight of the third mixture. The amount of diluent to be optionally added is comprised between 5% and 30% by weight with respect to the total weight of the third mixture, preferably between 7% and 25% of the total weight. The amount of binder to be optionally added to the mixture obtained in step c) is comprised between 1% and 15% by weight with respect to the total weight of the third mixture.

In step d) the first mixture, the second mixture, and optionally the third mixture are blended together. The first, second and third mixture can be in form of granules, coated granules or powder, with the proviso, that the first and second mixture can not be in form of powder at the same time.

In step e) the blend of step d) is filled into capsules or is compressed into tablets. Optionally, the tablets thus obtained are coated with a film coating agent.

The solid oral compositions may be prepared by conventional methods of blending, filling or tabletting. Repeated blending operations may be used to distribute the active agents throughout those compositions employing large quantities of fillers. Such operations are conventional in the art. The tablets may for example be prepared by wet dry or hot melt granulation and optionally coated according to methods well known in normal pharmaceutical practice, in particular with an enteric coating.

The mentioned formulations will be prepared using standard methods such as those described or referred to in the European and US Pharmacopoeias and similar reference texts.

In a preferred embodiment of the invention, the pharmaceutical composition is in the form of a tablet. The excipients used for preparing tablets may comprise between 0.5% and 4% by weight with respect to the total weight of the tablet of one or more lubricating agents, between 1% and 20% by weight of one or more disintegrants, between 5% and 50% by weight of one or more diluents and between 0.1% and 2% by weight of a glidant.

As disintegrant, sodium starch glycollate, low-substituted hydroxypropylcellulose, hydroxyethylcellulose, crospovidone, crosscarmellose, starch, sodium carboxymethyl starch, casein derivatives or mixture thereof can be used.

As lubricating agent, magnesium stearate, calcium stearate, glyceryl palmitostearate, talcum, stearic acid, glyceryl behenate, sodium lauryl sulfate, sodium stearyl fumarate, sucrose fatty acid ester or mixtures thereof can be used. A stearate will preferably be used, still more preferably, magnesium stearate.

As diluent, a saccharide (monosaccharide or oligosaccharide, polysaccharides) and/or their oxidized and/or reduced forms; lactose in its anhydrous, monohydrate, agglomerated or spray forms; mannitol; cellulose powder, microcrystalline cellulose, silicified microcrystalline cellulose or chemically modified cellulose derivatives, such as hydroxypropylcellulose, hydroxypropylmethylcellulose; starch, sucrose, maltodextrins, pharmaceutically acceptable inorganic compounds such as dibasic calcium phosphate, calcium or magnesium carbonates, magnesium oxide, or mixtures thereof can be used.

Additionally, previously prepared coprocessed diluents can be used such as Cellactose®, coprocessed lactose and cellulose powder, or Microcellac®, coprocessed lactose and microcrystalline cellulose, among others.

As glidant, anhydrous or hydrated colloidal silica, silicon dioxide colloidal anhydrous, magnesium trisilicate or talc can be used.

Other excipients that improve the compression of the final mixture, such as maltodextrines, can be added.

In the following, the present invention is further illustrated by examples. They should in no case be interpreted as a limitation of the scope of the invention as defined in the claims.

### EXAMPLES

### Example 1.

| | **mg per tablet** | **mg per tablet** | **mg per tablet** | **mg per tablet** |
|---|---|---|---|---|
| 1.Entacapone | 200 | 200 | 200 | 200 |
| 2.Levodopa | 30 | 30 | 30 | 30 |
| 3.Povidone | 19.5 | 19.5 | 19.5 | 19.5 |
| 4.Crospovidone | 9.8 | 9.8 | 9.8 | 9.8 |
| 5.Lactose monohydrate | 24.2 | 24.2 | 24.2 | 24.2 |
| **Entacapone-levodopa granules total** | **283.5** | **283.5** | **283.5** | **283.5** |
| | | | | |
| 6.Levodopa | 20 | 70 | 120 | 170 |
| 7.Carbidopa monhydrate | 13.5 | 27 | 40.5 | 54 |
| 8.Povidone | 4.2 | 11.8 | 19.5 | 27.3 |
| 9.Crospovidone | 2.1 | 5.9 | 9.8 | 13.7 |
| 10.Lactose monohydrate | 5.2 | 14.8 | 24.2 | 34.0 |
| **Levodopa-carbidopa granules total** | **45** | **129.5** | **214** | **299** |
| | | | | |
| 11.Lactose monohydrate | 75.5 | 94.6 | 113.4 | 132.2 |
| 12.Crospovidone | 8.7 | 10.8 | 13 | 15.1 |
| 13.Hydroxypropylcellulose | 10.8 | 13.5 | 16.3 | 18.9 |
| 14.Magnesium stearate | 6.5 | 8.1 | 9.8 | 11.3 |
| **Total core weight** | **430** | **540** | **650** | **760** |
| | | | | |
| 15. HPMC base coating agent | 13 | 16 | 20 | 23 |
| **Total tablet weight** | **443** | **556** | **670** | **783** |

### Example 2.

### Granules of Entacapone-levodopa are prepared as in example 1.

| | **mg per tablet** | **mg per tablet** | **mg per tablet** | **mg per tablet** |
|---|---|---|---|---|
| 6.Levodopa | 20 | 70 | 120 | 170 |
| 7.Carbidopa monhydrate | 13.5 | 27 | 40.5 | 54.0 |
| 8.Povidone | 4.2 | 11.8 | 19.5 | 27.3 |
| 9.Crospovidone | 2.1 | 5.9 | 9.8 | 13.7 |
| 10.Cellulose microcrystalline | 5.2 | 14.8 | 24.2 | 34.0 |
| **Levodopa-carbidopa granules total** | **45** | **129.5** | **214** | **299** |
| | | | | |
| 11.Lactose monohydrate | 75.5 | 94.6 | 113.4 | 132.2 |
| 12.CrosscarmelloseCrosscarmellose sodium | 8.7 | 10.8 | 13 | 15.1 |
| 13. Low-substituted Hydroxypropylcellulose | 10.8 | 13.5 | 16.3 | 18.9 |
| 14.Magnesium stearate | 6.5 | 8.1 | 9.8 | 11.3 |
| **Total core weight** | **430** | **540** | **650** | **760** |
| | | | | |
| 15. Triacetin base coating agent | 13 | 16 | 20 | 23 |
| **Total tablet weight** | **443** | **556** | **670** | **783** |

### Example 3.

| | **mg per tablet** | **mg per tablet** | **mg per tablet** | **mg per tablet** |
|---|---|---|---|---|
| 1.Entacapone | 200 | 200 | 200 | 200 |
| 2.Levodopa | 30 | 30 | 30 | 30 |
| 3.HPMC (Hydroxypropylmethyl | 19.5 | 19.5 | 19.5 | 19.5 |
| cellulose) | | | | |
| 4.Crosscarmellose | 9.8 | 9.8 | 9.8 | 9.8 |
| 5.Cellulose | 24.2 | 24.2 | 24.2 | 24.2 |
| **Entacapone-levodopa granules total** | **283.5** | **283.5** | **283.5** | **283.5** |
| | | | | |
| 6.Levodopa | 20 | 70 | 120 | 170 |
| 7.Carbidopa monhydrate | 13.5 | 27 | 40.5 | 54 |
| 8.HPMC | 4.2 | 11.8 | 19.5 | 27.3 |
| 9.Crosscarmellose | 2.1 | 5.9 | 9.8 | 13.7 |
| 10.Maize Starch | 5.2 | 14.8 | 24.2 | 34.0 |
| **Levodopa-carbidopa granules total** | **45** | **129.5** | **214** | **299** |
| | | | | |
| 11.Anhydrous lactose | 66.8 | 83.6 | 100.4 | 116.8 |
| 12.Crosscarmellose | 8.7 | 10.8 | 13 | 15.1 |
| 13.Maltodextrines | 19.5 | 24.4 | 29.3 | 34.1 |
| 14.Magnesium stearate | 6.5 | 8.1 | 9.8 | 11.3 |
| **Total core weight** | **430** | **540** | **650** | **760** |
| | | | | |
| 15. HPMC base coating agent | 13 | 16 | 20 | 23 |
| **Total tablet weight** | **443** | **556** | **670** | **783** |

### Manufacturing process (examples 1 ,2 and 3):

a) Component 1 is mixed with compounds 2, 3, 4 and 5. The mixture is granulated in a suitable mixer with purified water, dried and sieved.
b) Component 6 is mixed with compounds 7, 8, 9 and 10. The mixture is granulated in a suitable mixer with purified water, dried and sieved.
c) Mix the granules obtained in both steps a) and b) together with the compounds 11, 12 and 13.
d) Add compound 14 to the mixture obtained in c)
e) The mixture of step d) can be used indistinctly to fill hard capsules or to be compressed in a tabletting machine.
f) The obtained cores are coated with a water suspension of the component 15.

### Example 4.

| | **mg per tablet** | **mg per tablet** | **mg per tablet** | **mg per tablet** |
|---|---|---|---|---|
| 1.Entacapone | 200 | 200 | 200 | 200 |
| 2.Levodopa | 30 | 30 | 30 | 30 |
| 3.HPMC | 19.5 | 19.5 | 19.5 | 19.5 |
| 4.Crospovidone | 9.8 | 9.8 | 9.8 | 9.8 |
| 5.Lactose monohydrate | 24.2 | 24.2 | 24.2 | 24.2 |
| **Entacapone-levodopa granules total** | **283.5** | **283.5** | **283.5** | **283.5** |
| 6.Glyceryl behenate | 9.8 | 9.8 | 9.8 | 9.8 |
| | | | | |
| 7.Levodopa | 20 | 70 | 120 | 170 |
| 8.Carbidopa monhydrate | 13.5 | 27 | 40.5 | 54 |
| 9.HPMC | 4.2 | 11.8 | 19.5 | 27.3 |
| 10.Maize starch | 7.3 | 20.7 | 24.0 | 13.7 |
| **Levodopa-carbidopa granules total** | **45** | **129.5** | **214** | **299** |
| 11.Glyceryl palmitostearate | 0.7 | 1.9 | 3.2 | 4.5 |
| | | | | |
| 12.Lactose monohydrate | 65 | 82.9 | 100.4 | 117.9 |
| 13.Crospovidone | 8.7 | 10.8 | 13 | 15.1 |
| 14.Hydroxypropylcellulose | 10.8 | 13.5 | 16.3 | 18.9 |
| 15.Magnesium stearate | 6.5 | 8.1 | 9.8 | 11.3 |
| **Total core weight** | **430** | **540** | **650** | **760** |
| | | | | |
| 16. HPMC base coating agent | 13 | 16 | 20 | 23 |
| **Total tablet weight** | **443** | **556** | **670** | **783** |

### Example 5.

| | **mg per tablet** | **mg per tablet** | **mg per tablet** | **mg per tablet** |
|---|---|---|---|---|
| 1.Entacapone | 200 | 200 | 200 | 200 |
| 2.Levodopa | 30 | 30 | 30 | 30 |
| 3.HPMC | 19.5 | 19.5 | 19.5 | 19.5 |
| 4.Crosscarmellose | 9.8 | 9.8 | 9.8 | 9.8 |
| 5.Cellulose | 24.2 | 24.2 | 24.2 | 24.2 |
| **Entacapone-levodopa granules total** | **283.5** | **283.5** | **283.5** | **283.5** |
| 6.Glyceryl behenate | 9.8 | 9.8 | 9.8 | 9.8 |
| | | | | |
| 7.Levodopa | 20 | 70 | 120 | 170 |
| 8.Carbidopa monhydrate | 13.5 | 27 | 40.5 | 54 |
| 9.HPMC | 4.2 | 11.8 | 19.5 | 27.3 |
| 10.Maize starch | 7.3 | 20.7 | 24.0 | 13.7 |
| **Levodopa-carbidopa granules total** | **45** | **129.5** | **214** | **299** |
| 11.Glyceryl palmitostearate | 0.7 | 1.9 | 3.2 | 4.5 |
| | | | | |
| 12.Anhydrous lactose | 56.3 | 71.9 | 87.4 | 102.5 |
| 13.Crosscarmellose | 8.7 | 10.8 | 13 | 15.1 |
| 14.Maltodextrines | 19.5 | 24.4 | 29.3 | 34.1 |
| 15.Magnesium stearate | 6.5 | 8.1 | 9.8 | 11.3 |
| **Total core weight** | **430** | **540** | **650** | **760** |
| | | | | |
| 16. HPMC base coating agent | 13 | 16 | 20 | 23 |

### Manufacturing process (examples 4 and 5):

a) Component 1 is mixed with compounds 2, 3, 4 and 5. The mixture is granulated in a suitable mixer with purified water, dried and sieved.
b) The obtained granules are coated with component 6.
c) Component 7 is mixed with compounds 8, 9 and 10. The mixture is granulated in a suitable mixer with purified water, dried and sieved.
d) The obtained granules are coated with component 11.
e) Mix the coated granules obtained in both steps b) and d) together with the compounds 12, 13 and 14.
f) Add compound 15 to the mixture obtained in e)
g) The mixture of step e) can be used indistinctly to fill hard capsules or to be compressed in a tabletting machine.
h) The obtained cores are coated with a water suspension of the component 16.

### Example 6.

Components 1 to 4 as in example 4. Component 5 is substituted as detailed below:

| | **mg per tablet** | **mg per tablet** | **mg per tablet** | **mg per tablet** |
|---|---|---|---|---|
| 5. Cellulose microcrystalline | 24.2 | 24.2 | 24.2 | 24.2 |
| **Entacapone-levodopa granules total** | **283.5** | **283.5** | **283.5** | **283.5** |
| 6.Glyceryl behenate | 13 | 13 | 13 | 13 |
| | | | | |
| 7.Levodopa | 20 | 70 | 120 | 170 |
| 8.Carbidopa monhydrate | 13.5 | 27 | 40.5 | 54 |
| 9. Hydroxypropylcellulose | 4.2 | 11.8 | 19.5 | 27.3 |
| 10.Crospovidone | 2.1 | 5.9 | 9.8 | 13.7 |
| 11.Maize starch | 5.2 | 14.8 | 24.2 | 34.0 |
| **Levodopa-carbidopa granules total** | **45** | **129.5** | **214** | **299** |
| | | | | |
| 12.Lactose monohydrate | 62.5 | 81.6 | 100.4 | 119.2 |
| 13.Crospovidone | 8.7 | 10.8 | 13 | 15.1 |
| 14.Hydroxypropylcellulose | 10.8 | 13.5 | 16.3 | 18.9 |
| 15.Magnesium stearate | 6.5 | 8.1 | 9.8 | 11.3 |
| **Total core weight** | **430** | **540** | **650** | **760** |
| | | | | |
| 16. PVA base coating agent | 13 | 16 | 20 | 23 |
| **Total tablet weight** | **443** | **556** | **670** | **783** |

### Example 7.

| | **mg per tablet** | **mg per tablet** | **mg per tablet** | **mg per tablet** |
|---|---|---|---|---|
| 1.Entacapone | 200 | 200 | 200 | 200 |
| 2.Levodopa | 30 | 30 | 30 | 30 |
| 3.HPMC | 19.5 | 19.5 | 19.5 | 19.5 |
| 4. Crosscarmellose | 9.8 | 9.8 | 9.8 | 9.8 |
| 5.Lactose monohydrate | 24.2 | 24.2 | 24.2 | 24.2 |
| **Entacapone-levodopa granules total** | **283.5** | **283.5** | **283.5** | **283.5** |
| 6.HPMC base coating agent | 13 | 13 | 13 | 13 |
| | | | | |
| 7.Levodopa | 20 | 70 | 120 | 170 |
| 8.Carbidopa monhydrate | 13.5 | 27 | 40.5 | 54 |
| 9.HPMC | 4.2 | 11.8 | 19.5 | 27.3 |
| 10.Crosscarmellose | 2.1 | 5.9 | 9.8 | 13.7 |
| 11.Lactose monohydrate | 5.2 | 14.8 | 24.2 | 34.0 |
| **Levodopa-carbidopa granules total** | **45** | **129.5** | **214** | **299** |
| | | | | |
| 12. Cellulose microcrystalline | 62.5 | 81.6 | 100.4 | 119.2 |
| 13. Crosscarmellose | 8.7 | 10.8 | 13 | 15.1 |
| 14.Hydroxypropylcellulose | 10.8 | 13.5 | 16.3 | 18.9 |
| 15.Magnesium stearate | 6.5 | 8.1 | 9.8 | 11.3 |
| **Total core weight** | **430** | **540** | **650** | **760** |
| | | | | |
| 16. HPMCbase coating agent | 13 | 16 | 20 | 23 |
| **Total tablet weight** | **443** | **556** | **670** | **783** |

### Manufacturing process (examples 6 and 7):

a) Component 1 is mixed with compounds 2, 3, 4 and 5. The mixture is granulated in a suitable mixer with purified water, dried and sieved.
b) The obtained granules are coated with component 6.
c) Component 7 is mixed with compounds 8, 9, 10 and 11. The mixture is granulated in a suitable mixer with purified water, dried and sieved.
d) Mix the coated granules obtained in steps b) and c) together with the compounds 12, 13 and 14.
e) Add compound 15 to the mixture obtained in d)
f) The mixture of step e) can be used indistinctly to fill hard capsules or to be compressed in a tabletting machine.
g) The obtained cores are coated with a water suspension of the component 16.

### Example 8.

| | **mg per tablet** | **mg per tablet** | **mg per tablet** | **mg per tablet** |
|---|---|---|---|---|
| 1.Entacapone | 200 | 200 | 200 | 200 |
| 2.Levodopa | 30 | 30 | 30 | 30 |
| 3.Maize starch | 53.5 | 53.5 | 53.5 | 53.5 |
| **Entacapone-levodopa granules total** | **283.5** | **283.5** | **283.5** | **283.5** |
| | | | | |
| 4.Levodopa | 20 | 70 | 120 | 170 |
| 5.Carbidopa monhydrate | 13.5 | 27 | 40.5 | 54 |
| 6.Maize starch | 11.5 | 32.5 | 53.5 | 75 |
| **Levodopa-carbidopa granules total** | **45** | **129.5** | **214** | **299** |
| | | | | |
| 7.Maltodextrin | 75.5 | 94.6 | 113.4 | 132.2 |
| 8.Crosscarmellose | 8.7 | 10.8 | 13 | 15.1 |
| 9.Hydroxypropylcellulose | 10.8 | 13.5 | 16.3 | 18.9 |
| 10.Magnesium stearate | 6.5 | 8.1 | 9.8 | 11.3 |
| **Total core weight** | **430** | **540** | **650** | **760** |
| | | | | |
| 11.PVA base coating agent | 13 | 16 | 20 | 23 |
| **Total tablet weight** | **443** | **556** | **670** | **783** |

### Manufacturing process:

a) Component 1 is mixed with compounds 2 and 3. The mixture is granulated in a suitable mixer with purified water, dried and sieved.
b) Component 4 is mixed with compounds 5 and 6. The mixture is granulated in a suitable mixer with purified water, dried and sieved.
c) Mix the granules obtained in both steps a) and b) together with the compounds 7, 8 and 9.
d) Add compound 10 to the mixture obtained in c)
e) The mixture of step d) can be used indistinctly to fill hard capsules or to be compressed in a tabletting machine.
f) The obtained cores are coated with a water suspension of the component 11.

### Example 9.

Granules of Entacapone-Levodopa are prepared as in example 4

| | **mg per tablet** | **mg per tablet** | **mg per tablet** | **mg per tablet** |
|---|---|---|---|---|
| 6.Levodopa | 20 | 70 | 120 | 170 |
| 7.Carbidopa monhydrate | 13.5 | 27 | 40.5 | 54 |
| 8.HPMC | 4.2 | 11.8 | 19.5 | 27.3 |
| 9.Crospovidone | 2.1 | 5.9 | 9.8 | 13.7 |
| 10.Lactose monohydrate | 5.2 | 14.8 | 24.2 | 34.0 |
| **Levodopa-carbidopa granules total** | **45** | **129.5** | **214** | **299** |
| | | | | |
| 11.Maltodextrin | 75.5 | 94.6 | 113.4 | 132.2 |
| 12.Crospovidone | 8.7 | 10.8 | 13 | 15.1 |
| 13.Hydroxypropylcellulose | 10.8 | 13.5 | 16.3 | 18.9 |
| 14.Sucrose fatty acid ester | 6.5 | 8.1 | 9.8 | 11.3 |
| **Total core weight** | **430** | **540** | **650** | **760** |
| | | | | |
| 15. HPMC base coating agent | 13 | 16 | 20 | 23 |
| **Total tablet weight** | **443** | **556** | **670** | **783** |

### Manufacturing process:

a) Component 1 is mixed with compounds 2, 3, 4 and 5. The mixture is granulated in a suitable mixer with purified water, dried and sieved.
b) Component 6 is mixed with compounds 7, 8, 9 and 10. The mixture is granulated in a suitable mixer with purified water, dried and sieved.
c) Mix the granules obtained in both steps a) and b) together with the compounds 11, 12 and 13.
d) Add compound 14 to the mixture obtained in c)
e) The mixture of step d) can be used indistinctly to fill hard capsules or to be compressed in a tabletting machine.
f) The obtained cores are coated with a water suspension of the component 15.

### Example 10.

Entacapone-Levodopa granules prepared as in example 1

| | **mg per tablet** | **mg per tablet** | **mg per tablet** | **mg per tablet** |
|---|---|---|---|---|
| 6.Levodopa | 15 | 30 | 45 | 60 |
| 7.Carbidopa monhydrate | 13.5 | 27 | 40.5 | 54 |
| 8.Povidone | 4.2 | 8.4 | 12.6 | 16.8 |
| 9.Crospovidone | 2.1 | 4.2 | 6.3 | 8.4 |
| 10.Lactose monohydrate | 5.2 | 10.4 | 15.6 | 20.8 |
| **Levodopa-carbidopa granules total** | **40** | **80** | **120** | **160** |
| | | | | |
| 11.Levodopa | 5 | 40 | 75 | 110 |
| 12.Lactose monohydrate | 75.5 | 99 | 122 | 148.8 |
| 13.Crospovidone | 8.7 | 12.5 | 16.5 | 19.3 |
| 14.Hydroxypropylcellulose | 10.8 | 16.9 | 23.2 | 27.1 |
| 15.Sucrose fatty acid ester | 6.5 | 8.1 | 9.8 | 11.3 |
| **Total core weight** | **430** | **540** | **650** | **760** |
| | | | | |
| 16. HPMC base coating agent | 13 | 16 | 20 | 23 |
| **Total tablet weight** | **443** | **556** | **670** | **783** |

### Manufacturing process:

a) Component 1 is mixed with compounds 2, 3, 4 and 5. The mixture is granulated in a suitable mixer with purified water, dried and sieved.
b) Component 6 is mixed with compounds 7, 8, 9 and 10. The mixture is granulated in a suitable mixer with purified water, dried and sieved.
c) Mix the granules obtained in both steps a) and b) together with the compounds 11, 12, 13 and 14.
d) Add compound 15 to the mixture obtained in c)
e) The mixture of step d) can be used indistinctly to fill hard capsules or to be compressed in a tabletting machine.
f) The obtained cores are coated with a water suspension of the component 16.

### Example 11.

| | **mg per tablet** | **mg per tablet** | **mg per tablet** | **mg per tablet** |
|---|---|---|---|---|
| 1.Levodopa | 20 | 70 | 120 | 170 |
| 2.Carbidopa monhydrate | 13.5 | 27.0 | 40.5 | 54 |
| 3.Povidone | 4.2 | 11.8 | 19.5 | 27.3 |
| 4.Crospovidone | 2.1 | 5.9 | 9.8 | 13.7 |
| 5.Lactose monohydrate | 5.2 | 14.8 | 24.2 | 34.0 |
| **Levodopa-carbidopa granules 45 total** | | **129.5** | **214** | **299** |
| | | | | |
| 6.Entacapone | 200 | 200 | 200 | 200 |
| 7.Levodopa | 30 | 30 | 30 | 30 |
| 8.Povidone | 30.3 | 33 | 35.8 | 38.4 |
| 9.Crospovidone | 18.5 | 20.6 | 22.8 | 24.9 |
| 10.Lactose anhydrous | 99.7 | 118.8 | 137.6 | 156.4 |
| 11.Sucrose fatty acid ester | 6.5 | 8.1 | 9.8 | 11.3 |
| **Total core weight** | **430** | **540** | **650** | **760** |
| | | | | |
| 12. PVA base coating agent | 13 | 16 | 20 | 23 |
| **Total tablet weight** | **443** | **556** | **670** | **783** |

### Manufacturing process:

a) Component 1 is mixed with compounds 2, 3, 4 and 5. The mixture is granulated in a suitable mixer with purified water, dried and sieved.
b) Mix the granules obtained in steps a) with the compounds 6, 7, 8, 9 and 10.
c) Add compound 11 to the mixture obtained in b)
d) The mixture of step c) can be used indistinctly to fill hard capsules or to be compressed in a tabletting machine.
e) The obtained cores are coated with a water suspension of the component 12.

### Example 12.

Entacapone-Levodopa granules prepared as in example 1

| | **mg per tablet** | **mg per tablet** | **mg per tablet** | **mg per tablet** |
|---|---|---|---|---|
| 6.Levodopa | 20 | 70 | 120 | 170 |
| 7.Carbidopa monhydrate | 13.5 | 27.0 | 40.5 | 54 |
| 8.Povidone | 15 | 25.3 | 35.8 | 46.2 |
| 9.Crospovidone | 10.8 | 16.7 | 22.8 | 28.8 |
| 10.Lactose monohydrate | 80.7 | 109.4 | 137.6 | 166.2 |
| 11.Magnesium stearate | 6.5 | 8.1 | 9.8 | 11.3 |
| **Total core weight** | **430** | **540** | **650** | **760** |
| | | | | |
| 12. HPMC base coating agent | 13 | 16 | 20 | 23 |
| **Total tablet weight** | **443** | **556** | **670** | **783** |

### Manufacturing process:

a) Component 1 is mixed with compounds 2, 3, 4 and 5. The mixture is granulated in a suitable mixer with purified water, dried and sieved.
b) Mix the granules obtained in steps a) with the compounds 6, 7, 8, 9 and 10.
c) Add compound 11 to the mixture obtained in b)
d) The mixture of step c) can be used indistinctly to fill hard capsules or to be compressed in a tabletting machine.
e) The obtained cores are coated with a water suspension of the component 12.

### Example 13.

Entacapone-Levodopa granules prepared as in example 1

| | **mg per tablet** | **mg per tablet** | **mg per tablet** | **mg per tablet** |
|---|---|---|---|---|
| 6.Levodopa | 15 | 30 | 45 | 60 |
| 7.Carbidopa monhydrate | 13.5 | 27 | 40.5 | 54 |
| 8.Povidone | 4.2 | 8.4 | 12.6 | 16.8 |
| 9.Crospovidone | 2.1 | 4.2 | 6.3 | 8.4 |
| 10.Lactose monohydrate | 5.2 | 10.4 | 15.6 | 20.8 |
| **Levodopa-carbidopa granules total** | **40** | **80** | **120** | **160** |
| | | | | |
| 11.Levodopa | 5 | 40 | 75 | 110 |
| 12.Lactose monohydrate | 1.6 | 12.5 | 23.4 | 35.2 |
| 13.Crospovidone | 0.2 | 1.7 | 3.2 | 4.4 |
| 14.Povidone | 0.4 | 2.9 | 5.4 | 8.8 |
| **Levodopa granules total** | **7.2** | **57.1** | **107** | **158.4** |
| 15. Lactose monohydrate | 74.4 | 89.2 | 103.9 | 117.3 |
| 16.Hydroxypropylcellulose | 10 | 12 | 14 | 16 |
| 17.Crospovidone | 8.4 | 10.1 | 11.8 | 13.5 |
| 18.Magnesiumstearate | 6.5 | 8.1 | 9.8 | 11.3 |
| **Total core weight** | **430** | **540** | **650** | **760** |
| | | | | |
| 19. HPMC coating agent | 13 | 16 | 20 | 23 |
| **Total tablet weight** | **443** | **556** | **670** | **783** |

### Manufacturing process:

a) Component 1 is mixed with compounds 2, 3, 4 and 5. The mixture is granulated in a suitable mixer with purified water, dried and sieved.
b) Component 6 is mixed with compounds 7, 8, 9 and 10. The mixture is granulated in a suitable mixer with purified water, dried and sieved.
c) Component 11 is mixes with compounds 12, 13 and 14. The mixture is granulated in a suitable mixer with purified water, dried and sieved.
d) Mix the granules obtained in the steps a) b) and c) together with the compounds 15, 16, and 17.
e) Add compound 18 to the mixture obtained in d)
f) The mixture of step e) can be used indistinctly to fill hard capsules or to be compressed in a tabletting machine.
g) The obtained cores are coated with a water suspension of the component 19.

## Claims

1. An oral solid pharmaceutical composition comprising entacapone, levodopa and carbidopa or pharmaceutically acceptable salts or hydrates thereof **characterized in that** it comprises:
a) A first mixture wherein 10 to 75% of the total amount of levodopa is mixed with entacapone, optionally in the presence of pharmaceutical acceptable excipients ;
b) A second mixture wherein from 25 to 90% of the total amount of levodopa is mixed with carbidopa, optionally in the presence of pharmaceutical acceptable excipients;
c) Optionally, a third mixture of remaining levodopa and pharmaceutical acceptable excipients.

2. The solid oral pharmaceutical composition of claim 1 wherein the ratio entacapone-levodopa in the mixture is from 1:0.05 to 1:0.75, preferably from 1:0.1 to 1:0.2.

3. The solid oral pharmaceutical composition of claims 1 to 2 wherein the mixture of levodopa and entacapone is in form of granules, or coated granules.

4. The oral solid composition of claims 1 to 2 wherein the mixture of levodopa and entacapone is in form of powder.

5. The composition of any of the preceding claims wherein the mixture of levodopa and carbidopa is in form of granules or coated granules.

6. The oral solid composition of claims 1 to 3 wherein the mixture of levodopa and carbidopa is in form of powder.

7. The solid oral pharmaceutical composition of claims 1 to 6, optionally containing an additional amount of levodopa separated from the mixtures of levodopa-entacapone and levodopa-carbidopa and pharmaceutical acceptable excipients.

8. The solid oral pharmaceutical composition of claim 7 wherein the additional amount of levodopa and excipients is in form of granules.

9. The solid oral composition according to any of the preceding claims in the form of a capsule.

10. The solid oral pharmaceutical composition of claims 1 to 8 in the form of a tablet.

11. The composition of claim 10 in the form of coated tablet.

12. A method for preparing a solid oral pharmaceutical composition according to claim 1, comprising the stages of:
a) providing a first mixture of entacapone or a pharmaceutically acceptable salt thereof and a substantial amount of levodopa and, optionally mixing it with excipients
b) providing a second mixture of levodopa and carbidopa or pharmaceutically acceptable salts thereof optionally together with excipients.
c) Optionally, mixing levodopa with excipients.
d) Mixing the mixtures obtained in steps a),b) and c)
e) Optionally adding extragranular excipients
f) Filling the mixture into a capsule or compressing into a tablet
g) Optionally, coating the tablet.

13. The method of claim 12 wherein levodopa and entacapone in step a) are added together in the form of granules.

14. The method of claim 12 wherein levodopa and carbidopa in step b) are added together in the form of granules.

15. The method of claim 12 wherein levodopa and excipients in step c) are added together in the form of granules.
